# EUROPÄISCHE PATENTSCHRIFT

(11) **EP 4 319 662 B1**
(45) Veröffentlichungstag und Bekanntmachung des Hinweises auf die Patenterteilung: **30.10.2024**
(21) Anmeldenummer: 22721024.2
(22) Anmeldetag: 06.04.2022
(51) Int. Cl.: A61B 17/28

(54) **MONTAGEVERFAHREN SOWIE SCHRAUBWERKZEUG UND SCHRAUBSET**
ASSEMBLY METHOD AND A SCREWDRIVING TOOL AND SCREW KIT
PROCÉDÉ D'ASSEMBLAGE ET OUTIL DE VISSAGE ET KIT DE VIS

(30) Priorität: 08.04.2021 DE 102021108715
(43) Veröffentlichungstag der Anmeldung: 14.02.2024
(73) Patentinhaber: Aesculap AG, 78532 Tuttlingen (DE)
(72) Erfinder: BARTHELMES, Sven, 78576 Emmingen-Liptingen (DE); MORALES, Pedro, 78532 Tuttlingen (DE)
(74) Vertreter: Winter, Brandl - Partnerschaft mbB
(86) Internationale Anmeldenummer: PCT/EP2022/059090
(87) Internationale Veröffentlichungsnummer: WO 2022/214535

(56) Entgegenhaltungen:
- FR-A1- 3 032 345
- US-A1- 2007 157 736

## Beschreibung

### Technisches Gebiet

Die vorliegende Offenbarung betrifft ein Montageverfahren zum Einschrauben einer Schraube in ein Innengewinde eines medizinischen Instruments, unter Verwendung eines Schraubwerkzeugs, um zwei Branchen des Instruments so miteinander zu verbinden, dass die zwei Branchen zueinander verschwenkbar sind. Zudem betrifft die vorliegende Offenbarung ein Schraubwerkzeug zur Durchführung eines solchen Montageverfahrens sowie ein Schraubset mit einem solchen Schraubwerkzeug und einer Schraube.

### Hintergrund der Erfindung

Zum Eindrehen einer Schraube in ein Innengewinde, etwa um dadurch zwei Branchen eines medizinischen Instruments fest miteinander oder insbesondere zueinander relativbeweglich, beispielsweise verschwenkbar zu verbinden, ist es erforderlich, ein Drehmoment auf die Schraube aufzubringen. Dieses Drehmoment kann insbesondere durch ein Schraubwerkzeug form- und/oder kraftschlüssig (d.h. reibschlüssig) auf die Schraube übertragen werden. Ein solches Verfahren zum Einschrauben einer Schraube ist beispielsweise aus der DE 100 41 576 A1 oder aus der US 2005/135 898 A1 bekannt. Alternativ können die zwei Branchen des Instruments durch Nieten verbunden werden, wie es beispielsweise aus der DE 698 09 500 T2 bekannt ist. Weitere Montageverfahren sind beispielsweise aus der US 2007/157736 A1 und aus der FR 3 032 345 A1 bekannt.

Beim Einschrauben der Schraube muss das durch das Schraubwerkzeug auf die Schraube übertragene Drehmoment eine zwischen der Schraube und dem Innengewinde wirkende Gewindereibkraft überwinden.

Zur kraftschlüssigen Drehmomentübertragung wird die Schraube mit einem Axialdruck/einer axialen Druckkraft durch das Schraubwerkzeug beaufschlagt, so dass eine zwischen dem Schraubwerkzeug und der Schraube wirkenden Flächenreibkraft entsteht und bei Drehung des Schraubwerkzeugs ein aus der Flächenreibkraft resultierendes Reibmoment auf die Schraube übertragen wird. Ist das Reibmoment zwischen Schraubwerkzeug und Schraube kleiner als ein durch die Gewindereibkraft resultierendes Moment, rutscht das Schraubwerkzeug durch und die Schraube wird nicht eingeschraubt. Dabei kann es zum Abrutschen des Schraubwerkzeugs und zu Beschädigungen an einer Oberfläche der Schraube oder eines das Innengewinde aufweisenden Bauteils kommen.

Je höher der durch das Schraubwerkzeug auf die Schraube aufgebrachte Axialdruck ist, desto größer sind die Flächenreibkraft und das resultierende, von dem Schraubwerkzeug an die Schraube übertragbare Reibmoment. Da jedoch der auf die Schraube aufgebrachte Axialdruck auch auf das Innengewinde wirkt und somit die Gewindereibkraft mit dem steigenden Axialdruck zunimmt, kann der Axialdruck nicht einfach beliebig erhöht werden, um das reibschlüssig von dem Schraubwerkzeug an die Schraube übertragbare Drehmoment zu erhöhen.

### Zusammenfassung der Offenbarung

Die dem Anmeldungsgegenstand zugrundeliegende Aufgabe wird durch ein erfindungsgemäße Methode mit den im Anspruch 1 angegebenen Merkmalen, und ein erfindungsgemäßes Instrument mit den im Anspruch 5 angegebenen Merkmalen, gelöst. Weitere Ausführungsformen sind in den Unteransprüchen angegeben. Es ist also die Aufgabe der vorliegenden Offenbarung, ein geeignetes Montageverfahren bereitzustellen, mit dem eine Schraube besonders einfach und zuverlässig eingeschraubt werden kann. Zudem sollen ein Schraubwerkzeug zur Durchführung des Montageverfahrens sowie ein Schraubset mit einem Schraubwerkzeug und einer Schraube bereitgestellt werden.

Die Aufgabe der vorliegenden Offenbarung wird durch ein Montageverfahren mit den Merkmalen des Patentanspruchs 1 sowie durch ein Schraubwerkzeug und ein Schraubset mit den Merkmalen der nebengeordneten Patentansprüche gelöst. Vorteilhafte Weiterbildungen sind Gegenstand der Unteransprüche.

Genauer gesagt dient das Montageverfahren zum Einschrauben einer Schraube in ein Innengewinde eines medizinischen Instruments unter Verwendung eines Schraubwerkzeugs, um zwei Branchen des Instruments so miteinander zu verbinden, dass die zwei Branchen zueinander verschwenkbar sind.

Das Montageverfahren weist die folgenden Schritte auf:
- Einprägen einer Stempelgeometrie in einen vorzugsweise unprofilierten Schraubenkopf der Schraube durch Aufbringen einer axialen Druckkraft auf die Schraube unter Verwendung des Schraubwerkzeugs selbst, und
- Drehen des Schraubwerkzeugs relativ zu dem Instrument um eine Schraubenlängsachse, um ein Drehmoment (insbesondere zur Einstellung eines Anzugsmoments oder einer Einschraubtiefe der Schraube) von dem Schraubwerkzeug (reibschlüssig sowie) formschlüssig über die eingeprägte Stempelgeometrie auf die Schraube zu übertragen und die Schraube in das Innengewinde einzuschrauben.

Der Kern der Offenbarung besteht demzufolge darin, dass der Schraubenkopf vor der Montage eine vorzugsweise unprofilierte, d.h. glatte, beispielsweise ebene oder gewölbte, formschlussangriffsflächenfreie Oberfläche hat. Insbesondere kann der Schraubenkopf als ein Rotationskörper ausgebildet sein, d.h. als ein rotationssymmetrischer Körper, der sich bei Drehung um jeden beliebigen Winkel auf sich selbst abbildet. Während der Montage wird die Stempelgeometrie in den Schraubenkopf eingeprägt, so dass der Schraubenkopf nach der Montage eine profilierte, d.h. reliefartige, Oberfläche besitzt, in der eine Vertiefung in Form der Stempelgeometrie ausgebildet ist, welche als Formschlussangriffsfläche beim Einschrauben der Schraube dienen kann. Beim Drehen des Schraubwerkzeugs greift die Stempelgeometrie des Schraubwerkzeugs in die eingeprägte Stempelgeometrie ein, so dass das Drehmoment von dem Schraubwerkzeug an die Schraube nicht nur reibschlüssig (durch das Aufbringen der axialen Druckkraft und das beim Drehen aus der Flächenreibkraft resultierende Reibmoment), sondern auch formschlüssig auf die Schraube übertragen wird.

Dies hat den Vorteil, dass das maximal übertragbare Drehmoment von dem Schraubwerkzeug an die Schraube erhöht werden kann. Dadurch, dass die Stempelgeometrie während der Montage durch das Schraubwerkzeug erzeugt, nämlich eingeprägt wird, entspricht die Vertiefung/Einprägung in dem Schraubenkopf exakt der Stempelgeometrie/Oberflächenform des Schraubwerkzeugs. Dies hat den Effekt, dass das Drehmoment formschlüssig, etwa "schlupffrei" bzw. "drehmomentverlustfrei", an die Schraube übertragen und die Schraube mit einem geringeren, durch das Schraubwerkzeug aufgebrachten Drehmoment in das Innengewinde eingeschraubt werden kann.

Gemäß einer bevorzugten Ausführungsform kann das Montageverfahren den folgenden Schritt aufweisen:
- Zentriertes Ausrichten des Schraubwerkzeugs und der Schraube beim Aufbringen der axialen Druckkraft auf die Schraube durch das Schraubwerkzeug.

Durch die zentrierte Ausrichtung kann eine geeignete Kraftübertragung gewährleistet werden.

Gemäß einer bevorzugten Ausführungsform kann das Montageverfahren den folgenden Schritt aufweisen:
- Axiales Aufeinander-Zubewegen eines Werkzeugschafts und eines Gegenhalters des Schraubwerkzeugs zum Einspannen der Schraube zwischen ihrem Schraubenkopf und ihrem Schraubenschaft in das Schraubwerkzeug, wobei die Stempelgeometrie durch das Einspannen in das Schraubwerkzeug eingeprägt wird und das Schraubwerkzeug mit der darin eingespannten Schraube zum Einschrauben der Schraube relativ zu dem Instrument gedreht wird.

Das heißt also, dass gemäß der bevorzugten Ausführungsform des Montageverfahrens die Schraube während der Montage axial in das Schraubwerkzeug eingespannt werden kann, so dass ein zwischen dem Schraubwerkzeug und der Schraube wirkende Axialdruck unter Umgehung des Innengewindes (und Vermeidung eines dadurch erhöhten Gewindereibmoments) vergrößert werden kann.

Insbesondere kann das Instrument so ausgebildet sein, dass eine erste Branche der beiden Branchen ein erstes Durchgangsloch aufweist und eine zweite Branche der beiden Branchen ein zweites Durchgangsloch aufweist, an dem das Innengewinde ausgebildet ist. Gemäß einer bevorzugten Ausführungsform kann das Montageverfahren den folgenden Schritt aufweisen:
- Axiales Bewegen des Gegenhalters auf den Werkzeugschaft zu und durch das zweite Durchgangsloch hindurch, bevor die Schraube in das Schraubwerkzeug eingespannt sowie in das Innengewinde eingesetzt wird, oder
- Hindurchführen der Schraube durch das erste Durchgangsloch hindurch und Einsetzen der Schraube in das Innengewinde, bevor der Gegenhalter durch das zweite Durchgangsloch hindurch auf den Werkzeugschaft axial zubewegt wird und die Schraube in das Schraubwerkzeug eingespannt wird.

Demnach kann die Schraube zuerst in das Schraubwerkzeug gespannt werden, bevor sie in das Innengewinde eingesetzt und eingeschraubt wird, oder die Schraube kann zuerst in das Innengewinde eingesetzt werden, bevor sie in das Schraubwerkzeug eingespannt und eingeschraubt wird.

Vorzugsweise kann die Schraube in ihrer eingeschraubten Position gesichert werden, beispielsweise durch Laserschweißen und/oder durch Klebstoff, der vor dem Einspannen in das Schraubwerkzeug aufgebracht werden kann.

Die Aufgabe der vorliegenden Offenbarung wird auch durch ein Schraubwerkzeug zur Durchführung eines beschriebenen Montageverfahrens gelöst, mit einem Werkzeugschaft, der einen endseitigen Schrauben-Eingriffsabschnitt hat, welcher zur Übertragung eines Drehmoments auf die Schraube vorgesehen und ausgebildet ist und der eine Eingriffsfläche hat, von der eine Stempelgeometrie hervorsteht, die vorgesehen und ausgebildet ist, um bei Übertragung einer axialen Druckkraft in Richtung zu der Schraube hin die Stempelgeometrie in die Schraube einzuprägen und ein Drehmoment von der Stempelgeometrie des Werkzeugschafts formschlüssig über die eingeprägte Stempelgeometrie an die Schraube zu übertragen.

Gemäß einer bevorzugten Ausführungsform kann die Eingriffsfläche des Werkzeugschafts konkav gekrümmt, insbesondere im Wesentlichen sphärisch sein. Vorzugsweise ist die Eingriffsfläche abgesehen von der Stempelgeometrie glatt/unprofiliert. Die Eingriffsfläche des Werkzeugschafts kann vorzugsweise derart vorgesehen und ausgebildet sein, dass ihre Form der Form des Schraubenkopfes im Wesentlichen entspricht. Das heißt also, dass der Schraubenkopf vorzugsweise eine im Wesentlichen konvex gekrümmte, insbesondere sphärische (abgesehen von der eingeprägten Stempelgeometrie unprofilierte) Oberfläche hat. Dadurch kann der Werkzeugschaft in vorteilhafter Weise geführt und zentriert ausgerichtet werden. Es erfolgt also bevorzugt eine Selbstzentrierung bei der Montage bzw. beim Aufbringen der axialen Druckkraft von dem Schraubwerkzeug auf den Schraubenkopf.

Gemäß einer bevorzugten Ausführungsform kann die Stempelgeometrie abgerundete Kanten haben. Dies hat den Vorteil, dass eine Bildung von scharfkantigen Spalten, Schlitzen, Taschen oder ähnlichem vermieden wird, da sich aus diesen Gewebereste, Schmutz und Körperflüssigkeiten bei einer Wiederaufbereitung des Instruments nicht zuverlässig und vollständig entfernen lassen. Ein weiterer Vorteil der abgerundeten/weichkantigen/gekrümmten Oberflächenausbildung der Stempelgeometrie liegt darin, dass Beschädigungen beim Abrutschen des Schraubwerkzeugs und somit eine eventuelle Nachbearbeitung der Schraube entfallen kann, die zur Gewährleistung einer für medizinische, insbesondere chirurgische Instrumente/Geräte geforderte Oberflächenqualität erforderlich ist.

Gemäß einer bevorzugten Ausführungsform kann das Schraubwerkzeug einen Gegenhalter haben, der derart axial zu dem Werkzeugschaft sowie gegenläufig zu dem Werkzeugschaft ausrichtbar oder ausgerichtet ist, dass dessen endseitiger Schrauben-Eingriffsabschnitt dem endseitigen Schrauben-Eingriffsabschnitt des Werkzeugschafts axial gegenüberliegt und dazu vorgesehen und ausgebildet ist, eine axiale Druckkraft in Richtung zu dem Werkzeugschaft hin auf die Schraube zu übertragen. Mit anderen Worten kann zusätzlich zu dem etwa stempelartigen Werkzeugschaft der etwa stempelartige Gegenhalter vorgesehen sein, die eine gemeinsame Längsachse haben und voneinander axial so beabstandet sind, dass der Schrauben-Eingriffsabschnitt des Werkzeugschafts dem Schrauben-Eingriffsabschnitt des Gegenhalters zugewandt ist. Somit kann die Schraube längsausgerichtet zwischen dem Werkzeugschaft und dem Gegenhalter angeordnet werden. Darüber hinaus ist der Gegenhalter entlang seiner Längsachse in Richtung zu dem Werkzeugschaft hin verlagerbar, so dass die axial in Richtung zu dem Werkzeugschaft hinwirkende Druckkraft auf die Schraube, und damit von der Schraube auf den Werkzeugschaft, übertragbar ist. Folglich entspricht eine zwischen dem Schrauben-Eingriffsabschnitt des Werkzeugschafts und der Schraube wirkende Kraft einer Summe der durch den Werkzeugschaft und den Gegenhalter jeweils in Richtung zu dem gegenüberliegenden/gegenläufigen Werkzeugschaft bzw. Gegenhalter gerichteten aufbringbaren Druckkraft. Somit kann die Schraube axial zwischen dem Werkzeugschaft und dem Gegenhalter eingespannt werden. Dies hat den Vorteil, dass der zwischen dem Schraubwerkzeug und der Schraube wirkende Axialdruck unter Umgehung des Innengewindes (und Vermeidung eines dadurch erhöhten Gewindereibmoments) vergrößert werden kann. Einfach gesagt wird die Normalkraft auf die Oberfläche der Schraube, insbesondere des Schraubenkopfs erhöht, während die auf die Schraube (als Ganzes) in Richtung zu dem Gegenhalter hinwirkende Axialkraft gleich bleibt bzw. durch die Druckkraft des Gegenhalters ausgeglichen wird.

Gemäß einer bevorzugten Ausführungsform kann die Stempelgeometrie eine Form eines Qualitätssymbols haben, beispielsweise als ein Buchstabe ausgebildet sein. Somit kann der Anwender erkennen, dass die Montage mit dem anmeldungsgemäßen Verfahren ausgeführt wurde und die Schraube mit einem vorbestimmten Drehmoment angezogen wurde.

Die vorliegende Offenbarung betrifft auch ein Schraubset aus einem beschriebenen Schraubwerkzeug und einer Schraube mit einem Schraubenkopf, dessen Oberfläche vorzugsweise unprofiliert ist und als eine Prägefläche für das Einprägen der Stempelgeometrie dient. Gemäß einer bevorzugten Ausführungsform kann der Schraubenkopf eine konvexe, vorzugsweise im Wesentlichen sphärische Form haben. Insbesondere kann der Schraubenkopf als ein Rotationskörper ausgebildet sein, d.h. als ein rotationssymmetrischer Körper, der sich bei Drehung um jeden beliebigen Winkel auf sich selbst abbildet.

Mit anderen Worten kann durch das anmeldungsgemäße Montageverfahren ein reinigungsoptimiertes Verbindungselement für chirurgische Instrumente bereitgestellt werden, bei dem das Problem gelöst wird, wonach sich in einem Schlitz der Schraube oder um den Schraubenkopf herum Gewebereste, Schmitz und Körperflüssigkeiten sammeln und anhaften können, die beim Aufbereitungsprozess nicht vollständig entfernt werden. Nachteile von bisherigen Verbindungselementen liegen unter anderem darin, dass sich der scharfkantig ausgeführte Schlitzgrund und Spalte nicht zuverlässig reinigen lassen, dass in der Massenproduktion nicht vermeidbare Montagefehler, wie das Abrutschen eines Schraubendrehers, durch manuell, etwa durch Polieren, nachbearbeitet werden müssen. Somit stellt sich die Aufgabe, eine Kombination aus einem Design eines Verbindungselementkopfs und einem geeigneten Montageverfahren zu ermöglichen, bei dem keine tiefen und am Grund scharfkantigen Taschen, Schlitze oder dergleichen vorhanden sind, aber gleichzeitig die Haltekräfte der gesicherten Verbindung gleich oder größer als bisher sind und die aufgebrachten Anzugsmomente/Montagekräfte fein einstellbar/dosierbar sind. Dabei können der Schraubenkopf und das Gewindeende sphärisch gestalten sein, so dass die sphärische Auflage das System führt und sich von selbst zentriert, wenn die Schraube beim Montageprozess zwischen ihrem Kopf und dem Gewindeende gespannt wird oder wenn das Schraubwerkzeug mit der axialen Druckkraft auf die Schraube gedrückt wird. Während des Einspannvorgangs wird eine definierte Geometrie in die Kontaktstelle der Schraube eingeprägt, welche zur form- und kraftschlüssigen Kraftübertragung genutzt wird. Dabei kann das Anzugsmoment oder der Gang bzw. das Widerstandsmoment der beweglichen Teile feinfühlig eingestellt und/oder über eine Sensorik gemessen und/oder protokoliert werden. Die Schraubverbindung kann in dieser Schraubenstellung, falls erforderlich, mittels Klebstoff, einem Laserschweißpunkt oder dergleichen gesichert werden. Da der Schraubenkopf ohne einen Schlitz oder ein ähnliches Element der Kraftübertragung (Formschlussmittel) vor der Montage ausgeführt ist, muss das Verbindungselement nicht nachbearbeitet werden, um die für chirurgische Instrumente/Geräte geforderte Oberflächenqualität zu erreichen. Zusammenfassend ergibt sich eine höhere mechanische Belastbarkeit, eine prozesssichere/reproduzierbare maschinelle Herstellbarkeit, eine Vereinfachung der Montage durch Erhöhung des Mechanisierungsgrads bei der Herstellung sowie bessere Reinigungseigenschaften.

### Kurzbeschreibung der Figuren

Fig. 1 zeigt eine perspektivische Darstellung eines Teils eines anmeldungsgemäßen Schraubwerkzeugs, das zur Durchführung eines anmeldungsgemäßen Montageverfahren vorgesehen ist;
Fign. 2 und 3 zeigen perspektivische Darstellungen einer mit dem Schraubwerkzeug einschraubbaren Schraube vor der Montage und eines Instruments, in das die Schraube mit dem anmeldungsgemäßen Montageverfahren eingeschraubt wurde;
Fign. 4 bis 6 zeigen weitere perspektivische Darstellungen des Schraubwerkzeugs zu verschiedenen Zeitpunkten des damit ausgeführten Montageverfahrens; und
Fig. 7 zeigt eine perspektivische Darstellung einer bevorzugten Ausführungsform des anmeldungsgemäßen Schraubwerkzeugs.

Nachstehend werden bevorzugte Ausführungsformen der vorliegenden Offenbarung auf der Basis der zugehörigen Figuren beschrieben.

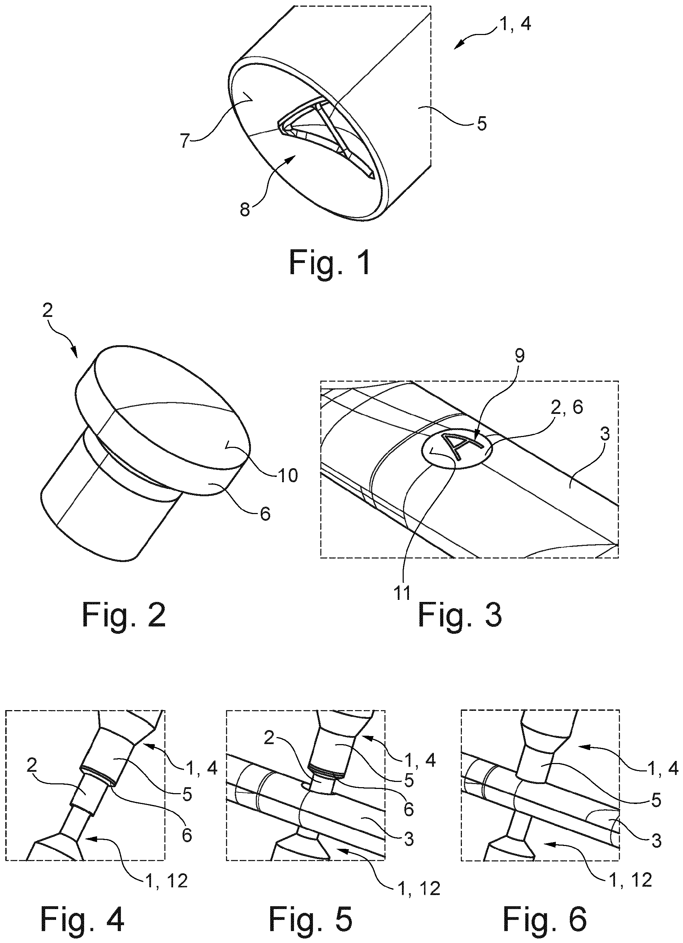

Fig. 1 zeigt eine perspektivische Darstellung eines Ausschnitts eines anmeldungsgemäßen Schraubwerkzeugs 1. Das Schraubwerkzeug 1 dient zum Einschrauben einer ein Außengewinde aufweisenden Schraube 2 in ein Innengewinde. Das Innengewinde ist in der dargestellten Ausführungsform an einem chirurgischen Instrument 3 ausgebildet. In den dargestellten Ausführungsformen legt die Schraube 2 zwei Bestandteile des Instruments 3 hier zueinander beweglich, insbesondere verschwenkbar fest.

Das Schraubwerkzeug 1 hat einen Werkzeugschaft 4 mit einem endseitigen Schrauben-Eingriffsabschnitt 5, der zur Übertragung eines Drehmoments auf die Schraube 2, insbesondere einen Schraubenkopf 6 der Schraube 2, vorgesehen und ausgebildet ist.

Der Schrauben-Eingriffsabschnitt 5 des Werkzeugschafts 4 hat eine Eingriffsfläche 7, von der eine Stempelgeometrie 8 hervorsteht, die vorgesehen und ausgebildet ist, um bei Übertragung einer axialen Druckkraft in Richtung zu der Schraube 2 hin die Stempelgeometrie 8 in die Schraube 2 (zur Erzeugung einer eingeprägten Stempelgeometrie 9) einzuprägen und ein Drehmoment von der Stempelgeometrie 8 des Werkzeugschafts 4 (reib- und) formschlüssig über die eingeprägte Stempelgeometrie 9 an die Schraube 2 zu übertragen.

Die Eingriffsfläche 7 kann vorzugsweise einer Form des Schraubenkopfes 6 entsprechen und insbesondere ein konkav gekrümmt sein. Vorzugsweise kann die Eingriffsfläche 7 sphärisch (oder kugelsegmentförmig) sein. Vorzugsweise ist die Eingriffsfläche 7 abgesehen von der Stempelgeometrie 8 glatt/unprofiliert.

Vorzugsweise kann die Stempelgeometrie 8 (und damit auch die eingeprägte Stempelgeometrie 9) abgerundete Kanten haben. Die Stempelgeometrie 8 kann eine Form eines Qualitätssymbols haben, beispielsweise als ein Buchstabe ausgebildet sein. In der dargestellten Ausführungsform weist die Stempelgeometrie 8 die Form eines "A"s auf.

Fig. 2 zeigt die Schraube 2, die mit dem anmeldungsgemäßen Montageverfahren in das Instrument 3 einschraubbar ist, vor der Montage. Insbesondere kann der Schraubenkopf 6 vor der Montage eine unprofilierte, d.h. glatte, beispielsweise ebene oder gewölbte, formschlussangriffsflächenfreie Oberfläche 10 haben. Die Oberfläche 10 dient als eine Prägefläche für das Einprägen der Stempelgeometrie 8. In der dargestellten Ausführungsform ist der Schraubenkopf 6 als ein Rotationskörper ausgebildet sein, d.h. als ein rotationssymmetrischer Körper, der sich bei Drehung um jeden beliebigen Winkel auf sich selbst abbildet. In der dargestellten Ausführungsform ist die Oberfläche 10 des Schraubenkopfes 6 konvex gewölbt.

Während der Montage wird die Stempelgeometrie 8 in den Schraubenkopf 6 (d.h. in die Oberfläche 10 des Schraubenkopfes 6) eingeprägt, so dass der Schraubenkopf 6 nach der Montage (vgl. Fig. 3) eine profilierte, d.h. reliefartige, Oberfläche 11 besitzt, in der eine Vertiefung in Form der Stempelgeometrie 8, d.h. die eingeprägte Stempelgeometrie 9, ausgebildet ist, welche als Formschlussangriffsfläche beim Einschrauben dienen kann. Beim Drehen des Schraubwerkzeugs 1 greift die Stempelgeometrie 8 des Schraubwerkzeugs 1 in die eingeprägte Stempelgeometrie 9 ein, so dass das Drehmoment von dem Schraubwerkzeug 1 an die Schraube 2 nicht nur reibschlüssig, sondern auch formschlüssig auf die Schraube 2 übertragen werden kann.

Vorzugsweise kann das Schraubwerkzeug 1 einen Gegenhalter 12 haben, der derart axial zu dem Werkzeugschaft 4 sowie gegenläufig zu dem Werkzeugschaft 4 ausrichtbar oder ausgerichtet ist, dass dessen endseitiger Schrauben-Eingriffsabschnitt dem endseitigen Schrauben-Eingriffsabschnitt 5 des Werkzeugschafts 4 axial gegenüberliegt und dazu vorgesehen und ausgebildet ist, eine axiale Druckkraft in Richtung zu dem Werkzeugschaft 4 hin auf die Schraube 2 zu übertragen (vgl. Fign. 4 bis 6). Dadurch kann die Schraube 2 in dem Schraubwerkzeug 1 zum Einprägen der Stempelgeometrie 8 und zum form- und reibschlüssigen Übertragen des Drehmoments vom dem Schraubwerkzeug 1 auf die Schraube 2 eingespannt werden.

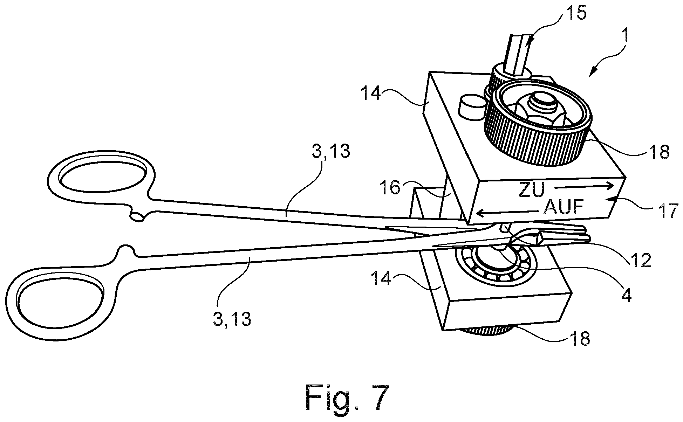

Die vorliegende Offenbarung betrifft auch ein Montageverfahren zum Einschrauben der Schraube 2 in das Innengewinde eines medizinischen Instruments 3, um zwei Branchen 13 des Instruments 3 so miteinander zu verbinden, dass die zwei Branchen 13 zueinander verschwenkbar sind. Das medizinische Instrument 3 und eine bevorzugte Ausführungsform ist in Fig. 7 näher dargestellt und wird später beschrieben.

In einem Schritt des Montageverfahrens wird die Stempelgeometrie 8 in einen vorzugsweise unprofilierten Schraubenkopf 6 (vgl. Fig. 2) der Schraube 6 durch Aufbringen einer axialen Druckkraft auf die Schraube 2 unter Verwendung des Schraubwerkzeugs 1 selbst eingeprägt. Dabei dient die vor der Montage unprofilierte Oberfläche 10 des Schraubenkopfs 6 als eine Prägefläche. In einem nachgelagerten Schritt des Montageverfahrens wird das Schraubwerkzeug 1 relativ zu dem Instrument 3 um eine Schraubenlängsachse gedreht, um ein Drehmoment (insbesondere zur Einstellung eines Anzugsmoments oder einer Einschraubtiefe der Schraube) von dem Schraubwerkzeug 1 formschlüssig über die eingeprägte Stempelgeometrie 9 auf die Schraube 2 zu übertragen und die Schraube 2 in das Innengewinde einzuschrauben (vgl. Fig. 3).

Vorzugsweise können das Schraubwerkzeug 1 und die Schraube 2 beim Aufbringen der axialen Druckkraft auf die Schraube 2 durch das Schraubwerkzeug 1 zentriert ausgerichtet werden. Die zentrierte Ausrichtung erfolgt insbesondere über die konkave Eingriffsfläche 8 und die konvex ausgebildete, unprofilierte Oberfläche 10 des Schraubenkopfes 6.

Vorzugsweise weist das Schraubwerkzeug den Gegenhalter 12 auf (vgl. Fign. 4 bis 6). Insbesondere können der Werkzeugschaft 4 und der Gegenhalter 12 des Schraubwerkzeugs 1 zum Einspannen der Schraube 2 zwischen ihrem Schraubenkopf 6 und ihrem Schraubenschaft in das Schraubwerkzeug 1 aufeinander zubewegt werden. Dabei wird die Stempelgeometrie 8 durch das Einspannen in das Schraubwerkzeug 1 eingeprägt und das Schraubwerkzeug 1 wird mit der darin eingespannten Schraube 2 zum Einschrauben der Schraube 2 relativ zu dem Instrument 3 gedreht.

Insbesondere kann das Instrument 3 so ausgebildet sein, dass eine erste Branche 13 der beiden Branchen 13 ein erstes Durchgangsloch aufweist und eine zweite Branche 13 der beiden Branchen 13 ein zweites Durchgangsloch aufweist, an dem das Innengewinde ausgebildet ist. Beispielsweise kann der Gegenhalter 12 auf den Werkzeugschaft 4 zu und durch das zweite Durchgangsloch hindurchbewegt werden, bevor die Schraube 2 in das Schraubwerkzeug 1 eingespannt sowie in das Innengewinde eingesetzt wird. Alternativ kann die Schraube 2 durch das erste Durchgangsloch hindurchgeführt und in das Innengewinde eingesetzt werden, bevor der Gegenhalter 12 durch das zweite Durchgangsloch hindurch auf den Werkzeugschaft 4 axial zubewegt wird und die Schraube 2 in das Schraubwerkzeug 1 eingespannt wird.

Vorzugsweise kann die Schraube 2 in ihrer eingeschraubten Position gesichert werden, beispielsweise durch Laserschweißen und/oder durch Klebstoff, der vor dem Einspannen in das Schraubwerkzeug 1 aufgebracht werden kann.

In Fig. 7 ist die bevorzugte Ausführungsform des Schraubwerkzeugs 1 dargestellt, in der das Schraubwerkzeug 1 den Werkzeugschaft 4 und den Gegenhalter 12 aufweist. Zudem sind in Fig. 7 die Branchen 13 des Instruments 3 dargestellt, die durch das anmeldungsgemäße Montageverfahren mittels der Schraube 2 so miteinander verbunden werden, dass die zwei Branchen 13 zueinander verschwenkbar sind.

Das Schraubwerkzeug 1 kann eine Halterung 14 aufweisen, an welcher der Werkzeugschaft 4 und/oder der Gegenhalter 12 angebracht sind. In der dargestellten Ausführungsform weist die Halterung 14 eine erste Aufnahme, an der der Werkzeugschaft 4 drehbar gelagert ist sowie eine zweite Aufnahme auf, an der der Gegenhalter 12 drehbar gelagert ist.

Das Schraubwerkzeug 1 kann zusätzlich eine Druckkraftvorrichtung 15 aufweisen. Die Druckkraftvorrichtung 15 ist dazu vorgesehen und ausgebildet, die Druckkraft (von dem Gegenhalter 12 axial in Richtung hin zum Werkzeugschaft 4 auf die Schraube 2 bzw. von dem Werkzeugschaft 4 axial in Richtung hin zum Gegenhalter 12 auf die Schraube 2) einzustellen, zu berechnen und/oder zu messen. Ein Axialabstand der beiden Aufnahmen ist über die Druckkraftvorrichtung 15 einstellbar. Die Halterung 14 weist eine Axialführung 16 auf, hier in Form von zwei parallel zu der Drehachse ausgerichteten Stangen, durch welche eine Einstellung des Axialabstands parallel zu der Drehachse geführt vorgegeben ist. An der Halterung 14 kann eine Skala 17 ausgebildet sein, die eine Ein-bzw. Ausdrehrichtung (close bzw. open) des Schraubwerkzeugs 1 indiziert.

Das Schraubwerkzeug 1 kann zusätzlich eine Anziehvorrichtung 18 aufweisen, die dazu vorgesehen und ausgebildet ist, einen Drehwinkel und/oder das Drehmoment des Werkzeugschafts 4 und/oder Gegenhalters 12 einzustellen und/oder zu messen. Die Anziehvorrichtung 18 kann manuell oder vorzugsweise automatisiert betätigbar sein. Die Anziehvorrichtung 18 kann in Form eines drehfest mit dem Werkzeugschaft 4 verbundenen Anziehrads bzw. eines drehfest mit dem Gegenhalter 12 verbundenen Anziehrads ausgebildet sein.

## Patentansprüche

1. Montageverfahren zum Einschrauben einer Schraube (2) in ein Innengewinde eines medizinischen Instruments (3), um zwei Branchen (13) des Instruments (3) so miteinander zu verbinden, dass die zwei Branchen (13) zueinander verschwenkbar sind, unter Verwendung eines Schraubwerkzeugs (1), **gekennzeichnet durch** die folgenden Schritte:
- Einprägen einer Stempelgeometrie (8) in einen vorzugsweise unprofilierten Schraubenkopf (6) der Schraube (2) durch Aufbringen einer axialen Druckkraft auf die Schraube (2) unter Verwendung des Schraubwerkzeugs (1) selbst, und
- Drehen des Schraubwerkzeugs (1) relativ zu dem Instrument (3) um eine Schraubenlängsachse, um ein Drehmoment von dem Schraubwerkzeug (1) formschlüssig über die eingeprägte Stempelgeometrie (9) auf die Schraube (2) zu übertragen und die Schraube (2) in das Innengewinde einzuschrauben.

2. Montageverfahren nach Anspruch 1, mit dem folgenden Schritt:
- Zentriertes Ausrichten des Schraubwerkzeugs (1) und der Schraube (2) beim Aufbringen der axialen Druckkraft auf die Schraube (2) durch das Schraubwerkzeug (1).

3. Montageverfahren nach Anspruch 1 oder 2, mit dem folgenden Schritt:
- Axiales Aufeinander-Zubewegen eines Werkzeugschafts (4) und eines Gegenhalters (12) des Schraubwerkzeugs (1) zum Einspannen der Schraube (2) in das Schraubwerkzeug (1), wobei die Stempelgeometrie (8) durch das Einspannen in das Schraubwerkzeug (1) eingeprägt wird und das Schraubwerkzeug (1) mit der darin eingespannten Schraube (2) zum Einschrauben der Schraube (2) relativ zu dem Instrument (3) gedreht wird.

4. Montageverfahren nach Anspruch 3, wobei eine erste Branche (13) der beiden Branchen (13) ein erstes Durchgangsloch aufweist und eine zweite Branche (13) der beiden Branchen (13) ein zweites Durchgangsloch aufweist, an dem das Innengewinde ausgebildet ist, mit dem folgenden Schritt:
- Axiales Bewegen des Gegenhalters (12) auf den Werkzeugschaft (4) zu und durch das zweite Durchgangsloch hindurch, bevor die Schraube (2) in das Schraubwerkzeug (1) eingespannt sowie in das Innengewinde eingesetzt wird, oder
- Hindurchführen der Schraube (2) durch das erste Durchgangsloch hindurch und Einsetzen der Schraube (2) in das Innengewinde, bevor der Gegenhalter (12) durch das zweite Durchgangsloch hindurch auf den Werkzeugschaft (4) axial zubewegt wird und die Schraube (2) in das Schraubwerkzeug (1) eingespannt wird.

5. Schraubwerkzeug (1) zur Durchführung eines Montageverfahrens nach einem der Ansprüche 1 bis 4, mit
einem Werkzeugschaft (4), der einen endseitigen Schrauben-Eingriffsabschnitt (5) hat, welcher zur Übertragung eines Drehmoments auf die Schraube (2) vorgesehen und ausgebildet ist, **dadurch gekennzeichnet, dass**
der Schrauben-Eingriffsabschnitt (5) des Werkzeugschafts (4) eine Eingriffsfläche (7) hat, von der eine Stempelgeometrie (8) hervorsteht, die vorgesehen und ausgebildet ist, um bei Übertragung einer axialen Druckkraft in Richtung zu der Schraube (2) hin die Stempelgeometrie (8) in die Schraube (2) einzuprägen und ein Drehmoment von der Stempelgeometrie (8) des Werkzeugschafts (4) formschlüssig über die eingeprägte Stempelgeometrie (9) an die Schraube (2) zu übertragen.

6. Schraubwerkzeug (1) nach Anspruch 5, **dadurch gekennzeichnet, dass** die Eingriffsfläche (7) konkav gekrümmt, insbesondere im Wesentlichen sphärisch ist.

7. Schraubwerkzeug (1) nach Anspruch 5 oder 6, **dadurch gekennzeichnet, dass** die Stempelgeometrie (8) abgerundete Kanten hat.

8. Schraubwerkzeug (1) nach einem der Ansprüche 5 bis 7, **dadurch gekennzeichnet, dass** das Schraubwerkzeug (1) einen Gegenhalter (12) hat, der derart axial zu dem Werkzeugschaft (4) sowie gegenläufig zu dem Werkzeugschaft (4) ausrichtbar oder ausgerichtet ist, dass dessen endseitiger Schrauben-Eingriffsabschnitt dem endseitigen Schrauben-Eingriffsabschnitt (5) des Werkzeugschafts (4) axial gegenüberliegt und dazu vorgesehen und ausgebildet ist, eine axiale Druckkraft in Richtung zu dem Werkzeugschaft (4) hin auf die Schraube (2) zu übertragen.

9. Schraubwerkzeug (1) nach einem der Ansprüche 5 bis 8, **dadurch gekennzeichnet, dass** die Stempelgeometrie (8) eine Form eines Qualitätssymbols hat, beispielsweise als ein Buchstabe ausgebildet ist.

10. Schraubset aus einem Schraubwerkzeug (1) nach einem der Ansprüche 5 bis 9 und einer Schraube (2) mit einem Schraubenkopf (6), dessen Oberfläche vorzugsweise unprofiliert ist und als eine Prägefläche für das Einprägen der Stempelgeometrie (8) dient.

## Claims

1. An assembly method for screwing a screw (2) into an internal thread of a medical instrument (3) in order to connect two branches (13) of the instrument (3) to each other such that the two branches (13) are pivotable relative to each other, using a screwdriving tool (1), **characterized by** the following steps:
- imprinting a punch geometry (8) into a preferably unprofiled screw head (6) of the screw (2) by applying an axial compression force to the screw (2) using the screwdriving tool (1) itself, and
- rotating the screwdriving tool (1) relative to the instrument (3) about a longitudinal screw axis to transmit torque from the screwdriving tool (1) in a form-fitting manner via the imprinted punch geometry (9) to the screw (2) and to screw the screw (2) into the internal thread.

2. The assembly method according to claim 1, comprising the following step:
- centered aligning of the screwdriving tool (1) and the screw (2) when the axial compression force is applied to the screw (2) by the screwdriving tool (1).

3. The assembly method according to claim 1 or 2, comprising the following step:
- axially moving a tool shaft (4) and a counterhold tool (12) of the screwdriving tool (1) for clamping the screw (2) into the screwdriving tool (1), wherein the punch geometry (8) is imprinted into the screwdriving tool (1) by the clamping and the screwdriving tool (1) with the screw (2) clamped therein is rotated relative to the instrument (3) for screwing in the screw (2).

4. The assembly method according to claim 3, wherein a first branch (13) of the two branches (13) comprises a first through-hole and a second branch (13) of the two branches (13) comprises a second through-hole at which the internal thread is configured, comprising the step of:
- axially moving the counterhold tool (12) towards the tool shaft (4) and through the second through-hole before clamping the screw (2) into the screwdriving tool (1) and inserting it into the internal thread, or
- passing the screw (2) through the first through-hole and inserting the screw (2) into the internal thread before moving the counterhold tool (12) axially through the second through-hole towards the tool shaft (4) and clamping the screw (2) into the screwdriving tool (1).

5. A screwdriving tool (1) for performing an assembly method according to one of claims 1 to 4, comprising
a tool shaft (4) having an end-side screw engagement portion (5) which is provided and configured for transmitting torque to the screw (2), **characterized in that**
the screw engagement portion (5) of the tool shaft (4) has an engagement surface (7) from which a punch geometry (8) protrudes which is provided and configured for imprinting the punch geometry (8) into the screw (2) upon transmission of an axial compression force toward the screw (2) and transmitting torque from the punch geometry (8) of the tool shaft (4) in a form-fitting manner via the imprinted punch geometry (9) to the screw (2).

6. The screwdriving tool (1) according to claim 5, **characterized in that** the engagement surface (7) is concavely curved, in particular substantially spherical.

7. The screwdriving tool (1) according to claim 5 or 6, **characterized in that** the punch geometry (8) has rounded edges.

8. The screwdriving tool (1) according to one of claims 5 to 7, **characterized in that** the screwdriving tool (1) has a counterhold tool (12) which is axially orientable or oriented with respect to the tool shaft (4) and in opposition to the tool shaft (4) such that its end-side screw engagement portion is axially opposed to the end-side screw engagement portion (5) of the tool shaft (4) and is provided and configured to transmit an axial compression force to the screw (2) in the direction towards the tool shaft (4).

9. The screwdriving tool (1) according to one of claims 5 to 8, **characterized in that** the punch geometry (8) has a form of a quality symbol, for example configured as a letter.

10. A screw kit comprising a screwdriving tool (1) according to one of claims 5 to 9 and a screw (2) having a screw head (6), the surface of which is preferably unprofiled and serves as an imprinting surface for imprinting the punch geometry (8).

## Revendications

1. Procédé de montage pour visser une vis (2) dans un filetage intérieur d'un instrument médical (3) pour connecter entre elles deux branches (13) de l'instrument (3), de sorte que les deux branches (13) puissent pivoter l'une par rapport à l'autre, en utilisant un outil de vissage (1), **caractérisé par** les étapes suivantes :
- estampage d'une géométrie de poinçon (8) dans une tête de vis (6), de préférence non profilée de la vis (2), en appliquant une force de pression axiale sur la vis (2) en utilisant l'outil de vissage (1) même, et
- rotation de l'outil de vissage (1) par rapport à l'instrument (3) autour d'un axe longitudinal de vis pour transmettre un couple de l'outil de vissage (1) à la vis (2) par complémentarité de formes par l'intermédiaire de la géométrie de poinçon (9) estampée et visser la vis (2) dans le filetage intérieur.

2. Procédé de montage selon la revendication 1 avec en outre l'étape suivante :
- alignement centré de l'outil de vissage (1) et de la vis (2) lors de l'application de la force de pression axiale sur la vis (2) par l'outil de vissage (1).

3. Procédé de montage selon la revendication 1 ou 2, avec l'étape suivante :
- déplacement axial l'un vers l'autre d'une tige d'outil (4) et d'un contre-support (12) de l'outil de vissage (1) pour serrer la vis (2) dans l'outil de vissage (1), dans lequel la géométrie de poinçon (8) est estampée dans l'outil de vissage (1) par le serrage, et l'outil de vissage (1) avec la vis (2) serrée dans celui-ci est tourné par rapport à l'instrument (3) pour visser la vis (2).

4. Procédé de montage selon la revendication 3, dans lequel une première branche (13) des deux branches (13) présente un premier trou traversant et une seconde branche (13) des deux branches (13) présente un second trou traversant au niveau duquel le filetage intérieur est formé, avec l'étape suivante :
- mouvement axial du contre-support (12) sur la tige d'outil (4) et à travers le second trou traversant avant que la vis (2) soit serrée dans l'outil de vissage (1) ainsi qu'insérée dans le filetage intérieur, ou
- passage de la vis (2) à travers le premier trou traversant et insertion de la vis (2) dans le filetage intérieur avant que le contre-appui (12) soit déplacé axialement sur la tige d'outil (4) à travers le second trou traversant et que la vis (2) soit serrée dans l'outil de vissage (1).

5. Outil de vissage (1) pour la mise en oeuvre d'un procédé de montage selon l'une quelconque des revendications 1 à 4, avec
une tige d'outil (4) qui présente une section d'engagement de vis (5) côté extrémité qui est prévue et conçue pour transmettre un couple à la vis (2), **caractérisé en ce que**
la section d'engagement de vis (5) de la tige d'outil (4) présente une surface d'engagement (7) de laquelle fait saillie une géométrie de poinçon (8) qui est prévue et conçue pour, lors de la transmission d'une force de pression axiale en direction de la vis (2), estamper la géométrie de poinçon (8) dans la vis (2) et transmettre un couple de la géométrie de poinçon (8) de la tige d'outil (4) à la vis (2) par complémentarité de formes par l'intermédiaire de la géométrie de poinçon estampée (9).

6. Outil de vissage (1) selon la revendication 5, **caractérisé en ce que** la surface d'engagement (7) est incurvée de manière concave, en particulier sensiblement sphérique.

7. Outil de vissage (1) selon la revendication 5 ou 6, **caractérisé en ce que** la géométrie de poinçon (8) présente des bords arrondis.

8. Outil de vissage (1) selon l'une quelconque des revendications 5 à 7, **caractérisé en ce que** l'outil de vissage (1) présente un contre-support (12) qui peut être orienté ou est orienté axialement par rapport à la tige d'outil (4) ainsi que dans le sens opposé à la tige d'outil (4), de sorte que sa section d'engagement de vis côté extrémité est axialement opposée à la section d'engagement de vis (5) de la tige d'outil (4) et est prévue et conçue pour transmettre une force de pression axiale à la vis (2) en direction de la tige d'outil (4).

9. Outil de vissage (1) selon l'une quelconque des revendications 5 à 8, **caractérisé en ce que** la géométrie de poinçon (8) présente une forme d'un symbole de qualité, par exemple une lettre.

10. Kit de vissage composé d'un outil de vissage (1) selon l'une quelconque des revendications 5 à 9 et d'une vis (2) avec une tête de vis (6) dont la surface est de préférence non profilée et sert de surface d'estampage pour l'estampage de la géométrie de poinçon (8).
